# EUROPEAN PATENT APPLICATION

(11) **EP 1 415 647 A1**
(43) Date of publication of application: **06.05.2004**
(21) Application number: 02023589.1
(22) Date of filing: 23.10.2002
(51) Int. Cl.: A61K 9/12, A61K 31/05, A61K 31/11, A61K 31/135, A61P 11/06, A61P 11/08

(54) **"Long-acting beta-2 agonists ultrafine formulations"**

(71) Applicant: CHIESI FARMACEUTICI S.p.A., I-43100 Parma (IT)
(72) Inventor: Church, Tanya Kathleen, 43100 Parma (IT); Lewis, David Andrew, 43100 Parma (IT); Ganderton, David, 43100 Parma (IT); Meakin, Brian, John, 43100 Parma (IT); Brambilla, Gaetano, 43100 Parma (IT)
(74) Representative: Minoja, Fabrizio, Dr.

(57) **Abstract**

The present invention relates to a pharmaceutical formulation for use in the administration of long-acting β₂-agonists by inhalation. In particular this invention relates to a highly efficient long acting β₂-agonist formulation to be administered by pressurised metered dose inhalers (pMDIs) characterized by a deeper lung penetration with a systemic exposure not significantly higher than that of the formulations currently on the market. The invention also relates to methods for their preparation and to their use in respiratory diseases such as asthma and chronic obstructive pulmonary disease (COPD).

## Description

### Background of the invention

Asthma is a disease which is becoming more prevalent and is the most common disease of childhood. It can be identified by recurrent wheeze and intermittent air flow limitation. Despite many advances in its understanding, said pathology remains a poorly understood and often poorly treated disease. Previously, contraction of airway smooth muscles has been regarded as the most important feature of asthma. Recently there has been a marked change in the way asthma is managed, stemming from the fact that asthma is recognized as a chronic inflammatory disease. Uncontrolled airway inflammation may lead to mucosal damage and structural changes giving irreversible narrowing of the airways and fibrosis of the lung tissue. Therapy should therefore be aimed at controlling symptoms so that normal life is possible and at the same time provide basis for treating the underlying inflammation.

Another respiratory disease whose incidence is steadily increasing throughout the world is chronic obstructive pulmonary disease (COPD). Most patients with COPD have acquired their lung disease through smoking cigarettes. Depending upon trends in tobacco smoking, it is set to rise to fifth most prevalent cause of disability, worldwide by 2020 (Leckie M et al *Exp Opin Invest Drugs* 2000, 9, 3-23).

Chronic obstructive pulmonary disease (COPD) is defined as a disease state characterized by the presence of airflow obstruction due to chronic bronchitis or emphysema.

Chronic bronchitis is characterized by excessive secretion of bronchial mucus, whereas emphysema denotes abnormal, permanent enlargement of air spaces distal to the terminal bronchiole, with destruction of their walls and without obvious fibrosis (American Toracic Society). Each condition is treated as specific diseases.

Chronic obstructive bronchiolitis is due to obstruction of the peripheral airways as a result of inflammation in the bronchioles.

β₂-Adrenoceptor agonists have been the mainstay of treatment for asthma for many years in view of their prompt bronchodilation effects. Previous researches have also shown that β₂-agonists have potent anti-inflammatory capabilities, e.g. represented by suppression of release of the pro-inflammatory cytokines.

The first generation drugs such as salbutamol or fenoterol were characterized by a relatively short duration of action which has been considered as a disadvantage particularly for patients with nocturnal asthma. Moreover, they have limited effects in COPD, since this disease involves "irreversible" airways obstruction. The development of longer acting β₂-agonists such as formoterol, salmeterol and TA 2005 has therefore been heralded as a major new development in the treatment of asthma. According to some authors, long-acting β₂-agonists (LABAs) may have acute anti-inflammatory activity in vivo (Johnson M *Clin Exp Allergy 1992, 22, 177-181;* Stelmach I et al *Ann Allergy Asthma Immunol 2002, 89, 67-73).* These drugs are a new interesting therapeutic option for patients with chronic obstructive pulmonary disease (COPD) as well since they have been shown to significantly improve lung function and symptom control.

Drugs intended for the treatment of lung diseases such as asthma are currently administered by pulmonary delivery which relies on inhalation of an aerosol through the mouth and throat so that the drug substance can reach the lung. They can be administered as aqueous or hydroalcoholic formulations through a nebuliser, as dry powders by means of Dry Powder Inhalers or in halogenated hydrocarbon propellants. The propellant-based systems require suitable pressurized metered-dose inhalers (pMDIs) which release a metered dose of medicine upon each actuation. The relevant formulations can be in the form of solutions or suspensions. Solution formulations, with respect to suspensions, do not present problems of physical stability of the suspended particles and so could guarantee a higher dose uniformity and reproducibility. As far as the type of propellant is concerned, hydrofluoroalkanes [(HFAs) known also as hydro-fluoro-carbons (HFCs)] would be mandatory propellants as chlorofluorocarbons (known also as Freons or CFCs), which were for many years the preferred propellants aerosols for pharmaceutical use, have been implicated in the destruction of the ozone layer so their use is being phased out. In particular, 1,1,1,2-tetrafluoroethane (HFA 134a) and 1,1,1,2,3,3,3-heptafluoropropane (HFA 227) have been acknowledged to be the best candidates for non-CFC propellants and a number of pharmaceutical aerosol formulations using such HFA propellant systems have been disclosed.

In developing a therapeutic aerosol, the aerodynamic size distribution of the inhaled particles is the most important variable in defining the site of droplet or particle deposition in the lungs of the patient; in short, it will determine whether drug targeting succeeds or fails. See P. Byron, "Aerosol Formulation, Generation, and Delivery Using Nonmetered Systems", Respiratory Drug Delivery, 144-151,144 (CRC Press, 1989).

Thus, a prerequisite in developing a therapeutic aerosol is a preferential particle size.

When the formulation is in the form of suspension, the particle size of the cloud is dominated by the particle size of the suspended drug, defined by the milling/micronization process. When the formulation is in the form of solution, the volumetric contribution of suspended drug particles is absent and much finer liquid droplets clouds, largely defined by the drug concentration in the solution, are generated.

Solid particles and/or droplets in an aerosol formulation can be characterized by their mass median aerodynamic diameter (MMAD, the diameter around which the mass aerodynamic diameters are distributed equally).

Particle deposition in the lung depends largely upon three physical mechanisms:
i) impaction, a function of particle inertia;
ii) sedimentation due to gravity; and
iii) diffusion resulting from Brownian motion of fine, submicrometer (< 1 microns) particles.

The mass of the particles determines which of the three main mechanisms predominates.

For aerosol therapy of drugs which topically act on the smooth muscle of the conducting airways, and in particular for β₂-agonists, it has been reported that particles should preferentially deposit in the upper-to mid-pulmonary region (bronchiole region), so they should have a MMAD of about 1.5 (2.0) to about 5.0 microns, preferably approximately 3 microns (Zanen P et al *Int J Pharm* 1994, 107, 211-217; *Int J Pharm* 1995, 114, 111-115; *Thorax,* 1996, 51, 977-980).

In fact, particles having aerodynamic diameters of greater than about 5 microns generally do not reach the lung since they tend to impact the back of the throat and are swallowed and possibly orally absorbed, while particles smaller than 1.5 (2.0) micron, i.e., about 0.5 to about 2 microns, are capable of reaching the alveolar region so they can be absorbed into the bloodstream and might enhance the undesired systemic effects of the drugs. Particles having diameters smaller than about 0.5 microns have been generally considered as not therapeutically useful as they can be exhaled.

Accordingly, pMDI formulations of β₂-agonist have traditionally been formulations able to deliver particles whose larger fraction is comprised between 2 and 5 microns and the amount of those below 1 micron is very limited since the former are small enough to reach the upper-to mid-pulmonary region, but are too large to reach the alveoli. This is also the inherent particle size of the formulation in the form of suspensions as conventional micronization (air-jet milling) of pure drug substance can reduce the drug particle size to about 2-3 microns.

On the other hand, it is known that the density of the beta-adrenergic receptors is higher in the distal tract of the bronchioles (Barnes P et al *Am Rev Respir Dis* 1983, 127, 758-762), a region which is better reached by smaller particles. Moreover inflammation in asthma in not merely confined to the large central airways but also extends to small peripheral airways. The eosinophilic inflammation process which has been seen to be associated to asthma concerns both the bronchial and the alveolar districts (Wang S *J Immunol* 2001, 166, 2741-2749). Distal lung diseases, in turn, appear to increase the risk of recurrent asthma exacerbation, whereas disease-related anatomic changes in the small airways of the distal lung are prominent in fatal asthma (Martin R *J Allergy Clin Immunol* 2002, 109 (Suppl 2), 447-460). Martin reports that the administration of drug particles with a diameter of about 1 micron (referred as "extrafine" aerosols) could be advantageous. The clinical significance of distal lung disease makes this region an important therapeutic target so particles able to reach and deposit into such region could better contribute to the management of the disease. It has been recently reported that, among the particles smaller than 0.5 micron, those with a diameter less or equal than 0.3 micron, preferably between 5 and 300 nm, can also be deposited in the alveolar region of the lung by sedimentation. This range of particle has been referred to in the literature as "ultrafine" particles.

"Ultrafine" particles generated from di-2-ethylhexyl sebacate (DEHS) as a model, have also been reported to have a good airway penetration (Anderson P et al *Chest* 1990, 97, 1115-1120). Therefore medicinal aerosol particles having a diameter < 0.1 µm can be particularly effective in case of airway obstruction in asthmatic subjects wherein the pathology is associated with mucus ipersecretion which hinders the diffusion of the drug or in patients affected by obstructive lung diseases such as COPD. Intuitively indeed, one would expect the reduction in the lumen of airways by mucus and permanent constriction would require finer clouds for perfusion.

In virtue of the inherent anti-inflammatory properties of LABAs, relevant formulations capable of delivering a significant fraction of fine particles would be expected to be of great advantage in patients affected by broncho-pulmonary obstructive diseases. Amirav I et al in *J Nucl Med 2002, 43, 487-491* emphasize the need for improvement in aerosol delivery by targeting narrow peripheral airways with superfine aerosols in the treatment of inflammation airways diseases and in particular in acute bronchiolitis.

In view of the above considerations, it would be highly advantageous to provide highly efficient long acting β₂-agonist formulation to be administered by pMDI characterized by a deeper lung penetration wherein, unexpectedly, the systemic exposure is not significantly higher than that of the formulations currently on the market.

### Description of the invention

The object of the present invention is to provide a pharmaceutical aerosol solution formulation to be administered by pMDI, having a suitable shelf-life for pharmaceutical use, comprising a long acting β₂-agonist, a HFA propellant, a suitable amount of co-solvent and optionally a proper amount of water wherein the active ingredient is completely dissolved in the propellant-co-solvent system. Said solution is able of providing on actuation of the formulation a fraction of particles less than 1.1 micron of at least 30% as defined by the content stages S6-AF of an Andersen Cascade Impactor relative to the fine particle dose (stages S3-AF).

The formulations of the invention, able to deliver a significant amount of particles having a diameter of about 1.0 micron (extrafine particles, according to Martin R *J Allergy Clin Immunol 2002, 109 (Suppl 2), 447-460*) and particles having a diameter equal or less than 0.3 micron (ultrafine particles, according to other authors) will be hereinafter referred to as superfine formulations.

In the prior art sub-micron aerosol formulations (including HFA formulations) have also been reported but in order to produce the nanoparticles for preparing the relevant formulations, surface active agents such as lecithin have been always used (WO 01/78689, WO 00/27363; Dickinson P et al *J Drug Target* 2001, 9, 295-302).

As a particular aspect of the present invention, we provide a pharmaceutical aerosol formulation comprising 0.003-0.096% w/v formoterol or one of its pharmaceutically acceptable salt such as fumarate as active ingredient in a solution of a liquefied HFA propellant and a co-solvent selected from the pharmaceutically acceptable alcohols characterized in that the fraction of particles less than 1.1 micron is higher or equal than 30% and the content of humidity as determined by Karl-Fischer method is less than 1500 ppm. In a preferred embodiment the pH of the formulation has been adjusted to between 2.5 and 5.0 as determined in the model vehicle system reported in EP 1157689. It has been surprisingly found that formoterol solution formulations with a significant fraction of particles below 1.1 micron have plasma levels in the earlier phase of absorption generally comparable to those of the CFC reference formulation on the market (Foradil) which has a small fraction of particles below 1.0 micron.

Even more surprisingly, it has even been found that the systemic exposure after one to two hours, which should correspond to the fraction of drug absorbed through the gut is slightly inferior to that of the reference formulation, making the formulation of the invention potentially better tolerated.

The present findings are contrary to what expected on what it is known for extrafine formulations comprising corticosteroids such as beclometasone dipropionate (BDP) or flunisolide. The latter ones indeed exhibit a very high systemic exposure which can produce side effects on bone metabolism and growth; so in order to develop formulations with the same therapeutic efficacy as those available on the market it is necessary to significantly lower the delivered dose (Donnell D Drug *Dev Ind Pharm* 2001, 27, 111-118; Harrison L et al *J Pharm Pharmacol* 1999, 51, 263-269; Demedts M et al *Int J Clin Practice* 1999, 53, 331-338; Corren J et al *Ann Allergy Asthma Immunol* 2001, 87, 405-411; Richards J et al *J Aerosol Med* 2001, 14, 197-208; Nolting A et al *Biopharm Drug Dispos* 2001, 22, 373-382).

A low systemic exposure of LABAs is particularly advantageous, since the plasma levels of the drug are responsible of side effects on the cardiovascular system.

As reported by the applicant in EP 1157689, through the adjustment of the apparent pH it is possible to dramatically improve the chemical stability of formoterol in HFA solution. The addition of a low amount of isopropyl myristate in order to increase the MMAD of the aerosol particles, may further improve the chemical stability of the compound.

It has now been found, as demonstrated in Example 3, that formoterol in the formulation is extremely sensitive to the residual humidity and for amount of water higher than 1500 ppm its content decreases to such a level which is not longer acceptable for pharmaceutical purposes (less than 90% w/w). The influence of a residual water content on the chemical stability of the active ingredient is particularly evident in high efficiency superfine formulations lacking of isopropyl myristate.

As another particular aspect of the present invention we provide formulations comprising 0.005-0.016% w/v TA 2005 or one of its pharmaceutically acceptable salt such as hydrochloride as active ingredient in a solution of a liquefied HFA propellant, a co-solvent selected from the pharmaceutically acceptable alcohols and optionally a proper amount of water characterized in that the fraction of particles less than 1.1 micron is higher or equal than 30% as defined by the content stages S6-AF of an Andersen Cascade Impactor relative to the fine particle dose.

In a preferred embodiment of the invention the pH of the formulation has been adjusted to between 2.5 and 5.0 as determined in the model vehicle system reported in EP 1157689.

It has indeed been found that the high efficiency of the formulation of the invention allows to utilise particularly low therapeutic doses (1-2 µg per shot) of such active ingredient which in view of its dose-dependent side effects is of noteworthy advantage (Burnet I et al *Eur Res J 1995, 8(Suppl 19), 258s*).

Moreover, since the ultrafine particles are reported to be retained for a longer time in the lung (Salvi S et al *Clin Exp All 1999, 29, 1197-1194*) and in virtue of the inherent longer duration of action of TA 2005 in comparison to other LABA's (Kikkawa H et al *Biol Pharm Bull* 1994, 17, 1047-1052), by means of the technology of the present invention it is possible to prepare TA 2005 formulations with a pharmaceutically acceptable shelf-life, well-tolerated and suitable for once daily administration with a great advantage in term of patient compliance.

The TA 2005 formulations could optionally contain water in an amount up to 1% w/w of the total weight of the formulation, preferably from 0.05 to 0.5%, more preferably from 0.1 to 0.3% w/w.

It has indeed been found that a proper amount of water can favourably affect the solubility of said active ingredient in the HFA:ethanol mixtures so the physical stability of the relevant formulations without increasing the MMAD of the aerosol droplets upon actuation.

As a further particular aspect of the invention we provide formulations comprising 0.02-0.05% w/v salmeterol or one of its pharmaceutically acceptable salt such as xinafoate as active ingredient in solution in a liquefied HFA propellant, a co-solvent selected from the pharmaceutically acceptable alcohols and a proper amount of water, characterized in that the fraction of particles less than 1.1 micron is higher or equal to 30% as defined by the content of stages S6-AF of an Andersen Cascade Impactor relative to the fine particle dose.

In the prior art, LABA solution formulations for aerosol delivery through pressurized metered-dose inhalers have been disclosed.

WO 94/13262 in the name of Boehringer Ingelheim provides aerosol solution formulations comprising a medicament, an HFC propellant, a co-solvent and an inorganic or an organic acid as a stabiliser for preventing the chemical degradation of the active ingredient. Most examples relate to ipratropium bromide, an anticholinergic drug. Although formoterol is cited among other active ingredients, no example is reported. Salmeterol and TA 2005 are not cited at all. As far as β₂-agonists are concerned, only formulations containing fenoterol are exemplified, a short acting derivative which is not chemically related to LABAs formoterol, salmeterol and TA 2005. Furthermore, apart from ipratropium bromide, in WO 94/13262 no guidance is given with respect to the amount of acid which has to be added in order to stabilise the medicaments without compromising the stability of the whole composition in the can. The only hint can be found on page 5, lines 15 to 16 which says that an amount of inorganic acid should be added to obtain a pH value from 1 to 7, so a very broad and generic range. As far as the water content is concerned, in the application it is stated that a small amount of water (up to about 5% by weight) may also be present in the propellant/co-solvent system. In the case of ipratropium bromide, it is reported that addition of 1% water reduces the decomposition due to dehydration. The document is silent about the effects of water on β₂-agonists and especially about the effect that an amount of residual water higher than 1500 ppm might have on the chemical stability of formoterol in solution in the propellant/co-solvent system.

WO 98/34596 in the name of 3 M refers to solution formulations containing a propellant and a physiologically acceptable polymer which could help the solubilisation and the stability as well of the active ingredients.

WO 98/34595 in the name of Jago Research refers to aerosol formulations in the form of solutions or suspensions in which the propellant is a mixture of a HFA and carbon dioxide. The presence of carbon dioxide can improve either physical and chemical stability of active compounds. LABAs such as formoterol and salmeterol are reported among the active compounds which can be used but no examples are reported.

WO 00/06121 in the name of Jago Research refers to propellant mixtures for aerosol containing dinitrogen monoxide and a hydrofluoroalkane in the preparation of suspension and solution aerosols. The use of dinitrogen monoxide may improve the stability at storage of oxidation-sensitive active ingredients. As far as LABAs such as formoterol fumarate and salmeterol xinafoate, only examples referred to suspensions are reported.

WO 99/65460 in the name of Baker Norton claims pressurised MDI's containing stable formulations of a β₂-agonist drug in suspension or solution. Examples refer to solutions of formoterol fumarate containing an HFA propellant and ethanol as a co-solvent, filled in conventional aluminium or plastic coated glass cans. Samples stored under accelerated conditions (40°C, 75% relative humidity) for a very short period, one month, exhibited about 10% loss of drug. According to pharmaceutical guidelines on stability, loss of 10% of active ingredient does not meet the criteria of acceptance. Moreover, even said document is silent about the dramatic effect of residual water on the chemical stability of formoterol and its salts.

In WO 98/56349 the applicant described solution compositions for use in an aerosol inhaler, comprising an active material, a propellant containing a hydrofluoroalkane (HFA), a co-solvent and further comprising a low volatility component to increase the mass median aerodynamic diameter (MMAD) of the aerosol particles on actuation of the inhaler. In some cases a small quantity of water may be added to the composition to improve the solution of the active material and/or the low volatility component in the co-solvent.

WO 01/37805 in the name of Glaxo is addressed to pharmaceutical aerosol formulations comprising salmeterol or a pharmaceutically acceptable salt thereof in a solution of a HFA propellant, a solubilising agent such as ethanol and a low volatility component to increase the MMAD of the aerosol particles on actuation of the inhaler as determined by the content of stages 3-5 of ACI. The formulations may preferably incorporate an agent in an amount capable of preventing chemical degradation of salmeterol, e.g. bases such as sodium or potassium hydroxide or sodium carbonate or an organic amine. It may be necessary also to incorporate a small quantity of water into the formulation e.g. 0.05-2% w/w water or more preferably 0.1-1% w/w water.

In the Examples, solutions of salmeterol in HFA and ethanol without low volatility component are reported as reference formulations. There is no mention of presence of water in these formulations.

The technical problem in WO 01/37805 is to match the particle size distribution of the suspension formulations containing CFC propellant, on the market.

Accordingly, the formulations contain a low-volatility component in order to increase the MMAD of the aerosol particles.

In EP 1157689 the applicant describes aerosol pharmaceutical compositions comprising a β₂-agonist belonging to the class of phenylalkylamino derivatives in solution in a HFA propellant, a co-solvent whose apparent pH has been adjusted to between 2.5 and 5.0 in order to guarantee an adequate shelf-life. In a particular embodiment of the invention, isopropyl myristate (IPM) as a low-volatility is added in order to either increase the MMAD of the aerosol particles and further improving the stability of the formulation. As far as the role of water is concerned, it is only generically stated that humidity, in the case of certain active ingredients such as formoterol, could be detrimental to the (chemical) stability during storage. As far as formoterol is concerned, HFA 134a formulations containing 12 w/w ethanol with or without 1.0% w/w IPM are reported in example 5. No guidance is given in EP 1157689 for further improving the stability of the relevant formulations by strictly controlling the residual amount of water, in particular when IPM, which improves the chemical stability of formoterol, is avoided. There is no preference in EP 1157689 for compositions containing IPM or not. On the other hand, in view of the knowledge of the prior art about the optimum particle size for β₂-agonists and the potential risks of side effects, the person skilled in the art would have utilized a low volatility component for matching the particle distribution of the formulation on the market.

In the case of TA 2005, formulations without the low volatility component are not described at all in EP 1157689. It is generically stated that TA 2005 formulations will be advantageously suitable for delivering 2-10 µg/dose, preferably 3-5 µg/dose. Only a 3.5 µg/dose HFA 134a formulation containing 12% w/w ethanol and 1.0% IPM is reported in example 7.

As mentioned above, the formulations of the invention can also comprise a further active ingredient. In particular, the addition of a corticosteroid to a long-acting β₂-agonist gives optimal control to asthma in most patients and relevant fixed combinations are increasingly used as a convenient controller in patients with persistent asthma. It has also been reported that each class of drug enhances the beneficial actions of the other. In fact, corticosteroids increase the expression of β₂-receptors and protect them against down-regulation in response to long-acting β₂-agonist exposure, whereas β₂-agonist may enhance the anti-inflammatory actions of corticosteroids (Barnes P et al. *Eur Respir J 2002,* 19, 182-191).

Accordingly, another object of the present invention is to provide highly efficient long-acting β₂-agonist formulations further comprising a steroid. The high fraction of superfine particles of the formulation of the invention can allow both drugs to reach the small peripheral airways region in such a way as to better exercise their synergic effects in distal lung diseases (*vide supra*). Moreover, in view of the aforementioned characteristics, it is possible to develop formulations comprising fixed combinations of a long-acting β₂-agonist and a steroid wherein the latter one could be present in a lower dose, by at the same maintaining the same therapeutic effect.

A further aspect of the present invention is to provide highly efficient long-acting β₂-agonist formulations further comprising an anticholinergic atropine-like derivative such as ipratropium bromide, oxitropium bromide and tiotropium bromide. This kind of combination has indeed been reported to be particularly effective for the treatment of COPD (D'Urzo et al *Chest, 2001, 119, 1347-1356*).

According to a further aspect of the invention there is provided a method of filling an aerosol inhaler with a composition of the invention, the method comprising:
(a) preparation of a solution of one or more active ingredients in one or more co-solvents
(b) optionally adding a pre-determined amount of water and/or adjusting the pH of the solution
(c) filling of the device with said solution
(d) crimping with valves and gassing
(e) adding a propellant containing a hydrofluoroalkane (HFA)

A still further aspect of the present invention comprises the use of the formulation of the invention comprising a long-acting β₂-agonist fully dissolved in the propellant/co-solvent system and capable of providing on actuation a fraction of at least 30% of emitted particles with an aerodynamic diameter of less than 1.1 microns, for the treatment of respiratory disorders such as asthma or COPD.

As reported in the literature, β₂-adrenergic agonists can stimulate alveolar fluid clearance in several animal species and in ex vivo rat and human lungs. In view of these findings beta-adrenergic agonist therapy has been proposed as a possible treatment for accelerating the resolution of pulmonary edema in patients with acute pulmonary edema (Sacuma T et al *Am J Respir Crit Care Med 1997, 155, 506-512).*

Treatment with β₂-agonists may also increase the secretion of surfactant and perhaps exert an anti-inflammatory effect, thus helping to restore vascular permeability of the lung (Ware L et al *New Eng. J Med* 2000, 342, 1334-1349. Accordingly, the formulation of the invention may be clinically useful as a treatment to hasten the resolution of alveolar edema and of surfactant-deficiency related diseases such as acute lung injury (ALI) and acute respiratory distress syndrome (ARDS).

Furthermore, in view of its technical feature of providing on actuation a fraction of particles with an aerodynamic diameter of less than 1.1 micron, of at least 30%, the formulation of the invention can be particularly effective for the treatment of airway obstruction conditions wherein the pathology is associated with mucus hypersecretion which hinders the diffusion of the drug.

### Detailed description of the invention

The aerosol formulations of the invention comprise an HFA propellant and a co-solvent wherein the active ingredient is fully dissolved in such a way that the formulations are able of providing on actuation a fraction of emitted particles of less than 1.1 microns higher or equal to 30% as defined by the content stages S6-AF of an Andersen Cascade Impactor relative to the respirable dose (stages S3-AF), advantageously higher than 40%, preferably higher than 50%, more preferably higher than 60%, even more preferably higher than 70%. Advantageously, the formulations of the invention are free of other excipients such as surfactants besides the solubilisation agent and the propellant.

Examples of HFA propellants include 1,1,1,2-tetrafluoroethane (HFA134a) and 1,1,1,2,3,3,3-heptafluoro-n-propane (HFA227) and mixtures thereof. The preferred propellant is 1,1,1,2-tetrafluoroethane (HFA134a). An alternative propellant of interest is 1,1,1,2,3,3,3-heptafluoro-n-propane (HFA227).

The co-solvent is usually an alcohol, preferably ethanol. When the presence of a residual content of water has to be avoided the co-solvent will be more preferably anhydrous ethanol, optionally dried on 3 A sieves. The concentration of the co-solvent (e.g. ethanol) will vary depending on the final concentration of the active ingredients in the formulation and on the propellant. The amount of ethanol should not exceed around 40% w/w of the total weight of the formulation. Advantageously it is comprised between 5 and 30% w/w, preferably between 10 and 20% w/w, even more preferably between 12 and 15% w/w.

Active ingredients which may be used in the aerosol compositions of the invention are long-acting β₂-adrenergic agonists (LABAs) such as formoterol, salmeterol, TA 2005, stereoisomers, salts and solvates thereof. Preferably, the active ingredient is a long acting β₂-agonists belonging to the formula sketched below 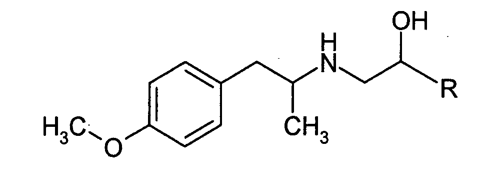 wherein R is more preferably 1-formylamino-2-hydroxy-phen-5-yl (formoterol) or 8-hydroxy-2(1H)-quinolinon-5-yl (TA 2005) or one of their corresponding stereoisomers.

In one of the embodiments of the invention, we prefer racemate formoterol to be used in the form of fumarate salt. In another one, we prefer the R,R-enantiomer of TA 2005 to be used in the form of hydrochloride salt.

Said active ingredients can be used alone or in combination with steroids such as beclometasone dipropionate (BDP), flunisolide, mometasone furoate, fluticasone propionate, budesonide and its 22R-epimer, with anticholinergic atropine-like derivatives such as ipratropium bromide, oxitropium bromide, tiotropium bromide or with drugs useful for the management of respiratory diseases such as methylxanthines, anti-leukotrienes and phosphodiesterase inhibitors.

The preferred combinations concern a long-acting β₂-agonist in combination with BDP or budesonide or its 22R-epimer.

The concentration of the LABA in the HFA formulation will depend on the therapeutic amount to be delivered preferably in one or two actuations.

In the foregoing drug concentrations are given as (w/v) and as fumarate salt for formoterol and as hydrochloride salt for TA 2005. The corresponding percentages as (w/w) can be calculated by determining the density of the vehicle.

In the case of formoterol, the concentration will vary between 0.003 and 0.096% w/v, preferably between 0.006 and 0.024% w/v in order to deliver 6 or 12 µg per actuation.

The formulation according to the invention will be used in association with a suitable metering valve. We prefer that the formulation is actuated by a metering valve capable of delivering a volume of between 25 µl and 100 µl, e.g. 50 µl or 63 µl. 100 µl is also suitable. For instance, for a 12 µg dose, when a 100 µl metering volume is used, the final concentration of formoterol fumarate delivered per actuation would be 0.012% w/v; wherein a 50 µl metering volume is used, the final concentration of formoterol fumarate would be doubled, e.g. 0.024% w/v; wherein a 63 µl metering volume is, which is the preferred one, the final concentration would be 0.019% w/v.

In the case of TA 2005 the concentration will vary between 0.0005 and 0.016% w/v, preferably between 0.001% and 0.004% w/v, in order to deliver 1 - 4 µg per actuation, especially 1 or 2 µg. If needed, a drug overage can be done. Even in this case, the formulations according to the invention will be used in association with a suitable metering valve. For instance, for 1 and 2 µg/dose, wherein a 63 µl metering volume is used, which is the preferred one, the final concentrations of TA 2005 hydrochloride delivered per actuation would be 0.0016% and 0.0032% w/v, respectively.

The apparent pH range is advantageously between 2.5 and 5.0, preferably between 3.0 and 4.5 for formoterol and between 2.8 and 4.0 for TA 2005. Strong mineral acids such as hydrochloric, nitric, phosphoric are preferably used to adjust the apparent pH.

The amount of acid to be added to reach the desired apparent pH will be pre-determined in the model vehicle reported in EP 1157689 and it will depend on the type and concentration of the active ingredient and the amount of the co-solvent. For 0.019% w/v formoterol fumarate solutions in HFA 134a and 12% w/w ethanol, an amount comprised between 3.85 and 4.85 µl of 1.0 M HCl is advantageously added, preferably between 4.15 and 4.55 µl of 1.0 M HCl, with the optimum of 4.35 µl. In more general terms, the concentration of 1.0 M HCl is between 0.030% w/w and 0.045% w/w, preferably between 0.035% and 0.040% w/w on the total weight of the formulation.

For 0.001-0.004% w/v TA 2005 hydrochloride in HFA 134a and 15% w/w ethanol, an amount between 1.5 and 2.5 µl (between 0.01% and 0.03% on the total weight of the formulation) of 0.1 M hydrochloric acid is preferably added. These formulations are able to give rise to a very high fraction of particles less than 1.1 micron as defined by the content stages S6-AF of an Andersen Cascade Impactor relevant to the fine particle dose.

The concentration of salmeterol expressed as weight of xinafoate will typically be in the range 0.02 - 0.05% w/v.

When the concentration of salmeterol xinafoate is around 0.05% w/v and the propellant is HFA 134a, an amount of ethanol around 30% w/w and an amount of water around 3% w/w are suitable. Said formulation is suitable for delivering 25 µg per actuation with a metering valve of 50 µl.

To improve the stability, it may be preferred to incorporate a base such as sodium or potassium hydrochloride or sodium carbonate or an organic amine.

The presence of water can be critical in certain formulations and will vary according to the characteristics of the active ingredient.

For instance, the amount of water in the formoterol fumarate formulations is lower than 1500 ppm, preferably lower than 1000 ppm, even more preferably lower than 500 ppm on the total weight of the formulation. It has been indeed found that in the case of certain TA 2005 hydrochloride formulations an amount of water up to 1.0% w/w on the total weight of the formulation might be advantageously present, preferably from 0.05 to 0.5 w/w.

For salmeterol xinafoate formulations an amount of water between 0.1% and 5% w/w, preferably between 1 and 3% w/w of the total weight of the formulation should be present.

The presence of such amounts of water is especially advantageous when the formulation further contains another active ingredient such as a steroid.

The formulations of the invention will be filled into canisters suitable for delivering pharmaceutical aerosol formulations such as plastic or plastic coated glass bottle or preferably a metal can, for example an aluminium can. More preferably, the formulations will be filled in canisters having part of all of the internal surfaces made of anodised aluminium, stainless steel or lined with an inert organic coating. Examples of preferred coatings are epoxy-phenol resins, perfluorinated polymers such as perfluoroalkoxyalkane, perfluoroalkoxyalkylene, perfluoroalkylenes such as poly-tetrafluoroethylene (Teflon), fluorinated-ethylene-propylene, polyether sulfone and a copolymer fluorinated-ethylene-propylene polyether sulfone. Other suitable coatings could be polyamide, polyimide, polyamideimide, polyphenylene sulfide or their combinations.

For example, canisters having the internal surface lined with Teflon might be preferred for the TA 2005 hydrochloride formulations.

To further improve the stability, cans having a rim with rounded edges, preferably a rolled neck or rolled-in rim, a part or full rollover rim are used according to the teaching of the co-pending application PCT/EP02/02710.

The canister is closed with a metering valve. The metering valves are designed to deliver a metered amount of the formulation per actuation and incorporate a gasket to prevent leakage of propellant through the valve.

The gasket may comprise any suitable elastomeric material such as for example low density polyethylene, chlorobutyl, black and white butadiene-acrylonitrile rubbers, butyl rubber, neoprene, EPDM (a polymer of ethylenepropylenediene monomer) and TPE (thermoplastic elastomer). EPDM and TPE rubbers are preferred. EPDM rubbers are particularly preferred. Suitable valves are commercially available from manufacturers well known in the aerosol industry, for example, from Valois, France (eg. DF10, DF30, DF60), Bespak plc, UK (eg. BK300, BK356, BK357) and 3M-Neotechnic Ltd, UK (eg. Spraymiser). The DF31 valve of Valois, France is also suitable. Valve seals, especially the gasket seal, and also the seals around the metering chamber, will preferably be manufactured of a material which is inert to and resists extraction into the contents of the formulation, especially when the contents include ethanol.

Valve materials, especially the material of manufacture of the metering chamber, will preferably be manufactured of a material which is inert to and resists distortion by contents of the formulation, especially when the contents include ethanol. Particularly suitable materials for use in manufacture of the metering chamber include polyesters eg polybutyleneterephthalate (PBT) and acetals, especially PBT.

Materials of manufacture of the metering chamber and/or the valve stem may desirably be fluorinated, partially fluorinated or impregnated with fluorine containing substances in order to resist drug deposition.

Conventional bulk manufacturing methods and machinery well known to those skilled in the art of pharmaceutical aerosol manufacture may be employed for the preparation of large scale batches for the commercial production of filled canisters. Thus, for example, in one bulk manufacturing method a metering valve is crimped onto an aluminum can to form an empty canister. The medicament is added to a charge vessel and a mixture of ethanol, optionally water and liquefied propellant is pressure filled through the charge vessel into a manufacturing vessel. An aliquot of the formulation is then filled through the metering valve into the canister.

In an alternative process, an aliquot of the liquefied formulation is added to an open canister under conditions which are sufficiently cold that the formulation does not vaporize, and then a metering valve crimped onto the canister.

In an alternative process, an aliquot of medicament dissolved in the solubilising agent is dispensed into an empty canister, a metering valve is crimped on, and then the propellant is filled into the canister through the valve. Preferably, the processes are carried out an in inert atmosphere, for instance by insufflating nitrogen, in order to avoid the uptake of humidity from the air.

Each filled canister is conveniently fitted into a suitable channeling device prior to use to form a metered dose inhaler for administration of the medicament into the lungs of a patient. Suitable channeling devices comprise, for example a valve actuator and a cylindrical or cone-like passage through which medicament may be delivered from the filled canister via the metering valve to the mouth of a patient e.g. a mouthpiece actuator.

In a typical arrangement the valve stem is seated in a nozzle block which has an orifice leading to an expansion chamber. The expansion chamber has an exit orifice which extends into the mouthpiece. Actuator (exit) orifice diameters in the range 0.15 - 0.45 mm especially 0.2 - 0.45 mm are generally suitable e.g. 0.25, 0.30, 0.33 or 0.42 mm. 0.22 mm is also suitable. For certain formulations it would be useful to utilize laser-drilled actuator orifices having a diameter ranging from 0.10 to 0.22 mm, in particular from 0.12 to 0.18 mm as those described in the co-pending application n. EP 1130521.6.

The use of such fine orifices also increases the duration of cloud generation and lowers its velocity. These changes facilitate the coordination of cloud generation with the slow inspiration of the patient. Thus slower clouds lead to the diffuse penetration of the areas into the lung.

In case the ingress of water into the formulation is to be avoided, it may be desired to overwrap the MDI product in a flexible package capable of resisting water ingress. It may also be desired to incorporate a material within the packaging which is able to adsorb any propellant and co-solvent which may leak from the canister (e.g. a molecular sieve).

The aerodynamic particle size distribution of each tested formulation of the invention can be characterized using a Multistage Cascade Impactor according to the procedure described in European Pharmacopoeia 2^{nd} edition, 1995, part V.5.9.1, pages 15-17. In this specific case, an Andersen Cascade Impactor (ACI) was utilized. Deposition of the drug on each ACI plate was determined by high pressure liquid chromatography (HPLC). Mean metered dose was calculated from the cumulative deposition in the actuator and ACI stages; mean delivered dose was calculated from the cumulative deposition in the ACI. Mean respirable dose (fine particle dose) was obtained from the deposition on Stages 3 (S3) to filter (AF) corresponding to particles ≤ 4.7 microns, divided by the number of actuation per experiment, while mean "superfine" dose was obtained from the deposition on Stages 6 to filter corresponding to particles ≤ 1.1 microns.

Administration of the formulations of the invention may be indicated for the treatment of mild, moderate or severe acute or chronic symptoms or for prophylactic treatment of respiratory diseases such as asthma and chronic obstructive pulmonary disease (COPD). Other respiratory disorders characterized by obstruction of the peripheral airways as a result of inflammation and presence of mucus such as chronic obstructive bronchiolitis and chronic bronchitis can also benefit by this kind of formulation.

The invention is illustrated with reference to the following examples.

### Example 1 Ultrafine formoterol HFA formulation

A formulation was prepared with the composition as follows:

| *Components* | *Amounts* | | |
|---|---|---|---|
| | Per unit | | Dose of a single actuation |
| | | % | µg |
| Formoterol fumarate | 1.92 mg | 0.019 w/v | 12 |
| Anhydrous ethanol | 1416.9 mg | 12 w/w | - |
| HCl 0.1 M | 4.40 mg* | 0.037 w/w | - |
| HFA 134a q.s. to 10.09 ml | | - | - |

| | | | |
|---|---|---|---|
| * equivalent to 4.35 µl | | | |

The formulation (120 actuations/canister, overage of 40 actuations) was filled in standard aluminum canisters (two stage pressure filling) under pressure and fitted with a metering valve having a 63 µl metering chamber. Two actuators were used: 0.30 and 0.42 mm. Results were obtained as a mean of 2-4 cans.

The aerodynamic particle size distribution was determined by ACI, according to page 23, lines 9-21.

The delivery characteristics of the formulation are reported in Table 1 in comparison with the reference CFC formulation currently available on the market (Foradil). In particular the following parameters are reported: i) nominal dose: theoretical dose per single actuation; ii) emitted dose: amount of active particles deposited into the various ACI stages; iii) respirable dose (fine particle dose): amount of active particles of size less than 4.7 microns (S3-AF); iv) respirable fraction (fine particle fraction): ratio between the respirable dose and the emitted dose; v) "superfine" dose: amount of active particles equal or less than 1.1 microns (S6-AF); iv) "superfine" fraction: ratio between the "superfine" dose and the respirable dose.

**Table 1 -**

| Delivery characteristics of the formoterol HFA solution formulations of the Ex. 1. | | | | | | |
|---|---|---|---|---|---|---|
| | Nominal Dose (µg) | Emitted dose (µg) | Respirable dose (µg) | Respirable fraction (%) | Superfine dose (µg) | Superfine Fraction (%) |
| Formulation Exl Act 0.30 mm | 12 | 10.02 | 3.31 | 32.5 | 2.53 | 76.4 |
| Formulation Ex 1 Act 0.42 mm | 12 | 10.84 | 2.14 | 19.7 | 1.57 | 73.3 |
| Foradil | 12 | 11.1 | 5.70 | 51.4 | 1.18 | 20.7 |

The results show that the formulations of the invention give rise upon actuation to a dramatically higher percentage of particles with a diameter less than 1.1 microns than the reference formulation.

In a preliminary clinical trial it was demonstrated that, thanks to their particles distribution, the formulations of Ex. 1 and 2 have a bronchodilator action equivalent to that of the reference formulation in CFC propellant.

### Example 2- Pharmacokinetics study

The aim of the study was to evaluate the pharmacokinetics of formoterol in 6 healthy volunteers after single administration of the formoterol formulations of Example 1 at 120 µg dose (10 shots x 12 µg/shot) in comparison with the marketed CFC formulation (Foradil). The experimental protocol is reported as follows:

### Treatments

- Foradil CFC 120 µg (10 shots x 12 µg/shot): Reference formulation
- Formoterol/HFA orifice 0.42 mm 120 µg (10 shots x 12 µg/shot): Test formulation
- Formoterol/HFA orifice 0.30 mm 120 µg (10 shots x 12 µg/shot): Test formulation

The study was a single dose cross-over study; subjects received the drug at 8 a.m. The wash-out among different treatments was of at least 1 weeks. Patients were instructed to take 10 doses. Time 0 for each dose was defined as the time when the MDI is first actuated.

### Bioanalysis

Assay of formoterol was carried out employing HPLC/MS validated method with a LOQ of 2pg/ml.

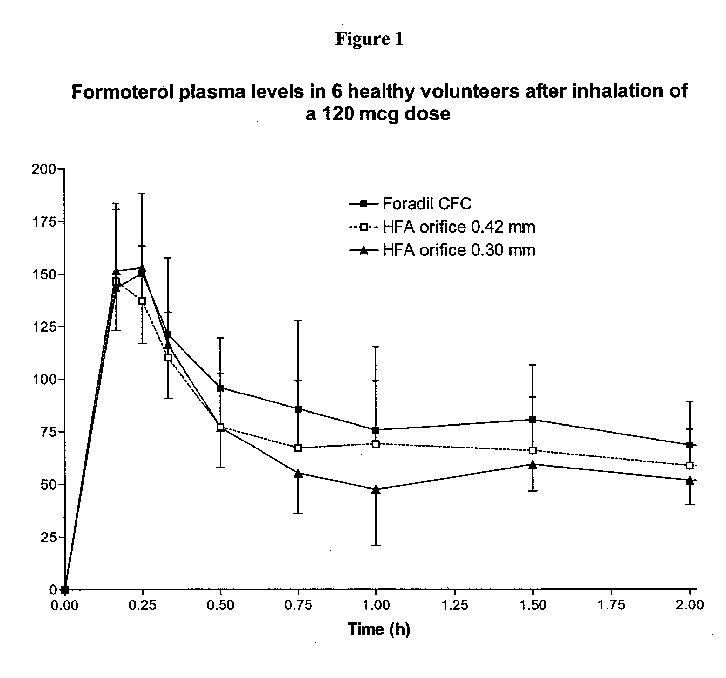

The pharmacokinetics parameters are reported in Table 2 while in Figure 1 the plasma concentration in the first two hours are shown.

**Table 2 -**

| Pharmacokinetics parameters | | | |
|---|---|---|---|
| | **Foradil CFC** | **Formoterol HFA of Ex.1 0.42 mm** | **Formoterol HFA of Ex.10 30 mm** |
| **Cmax** (pg ml⁻¹) | *159 ± 34* | *150 ± 36* | 158 ± 32 |
| **AUC(0- 20min)** (pgml⁻¹*h) | *35.4 ± 9.0* | *34.3 ± 7.3* | 36.5 ± 7.3 |
| **AUCt** (pgml⁻¹*h) | *655 ± 153* | *611 ± 103* | 578 ± 98 |
| Cₘₐₓ is the maximum plasma concentration. AUC_{0-20 min} is the area under the curve of the plasmatic levels from time 0 h to 20 minutes. AUCₜ is the area under the curve of the plasmatic levels from time 0 h to the last measurable data point. | | | |

The results demonstrate that the formoterol formulations of Example 1 having a significant fraction of particles below 1.1 µm show plasma levels in the first part of the curve (up to 20 min) comparable to those of the CFC reference formulation on the market (Foradil) which has a small fraction of particles below 1.0 µm. The systemic exposure, after one to two hours, which should correspond to the fraction of drug absorbed through the gut is slightly inferior to that of the reference formulation. Moreover the systemic exposure does not correlate with the total quantity of the fine particle dose.

### Example 3 - Effect of the residual humidity on the formoterol assay

The formulation of Example 1 filled in standard aluminum cans was stored in different conditions (25°C, 40°C) and for different times (0, 3, 6 months).

The assay of formoterol was determined by HPLC while the water content was determined by Karl-Fischer method.

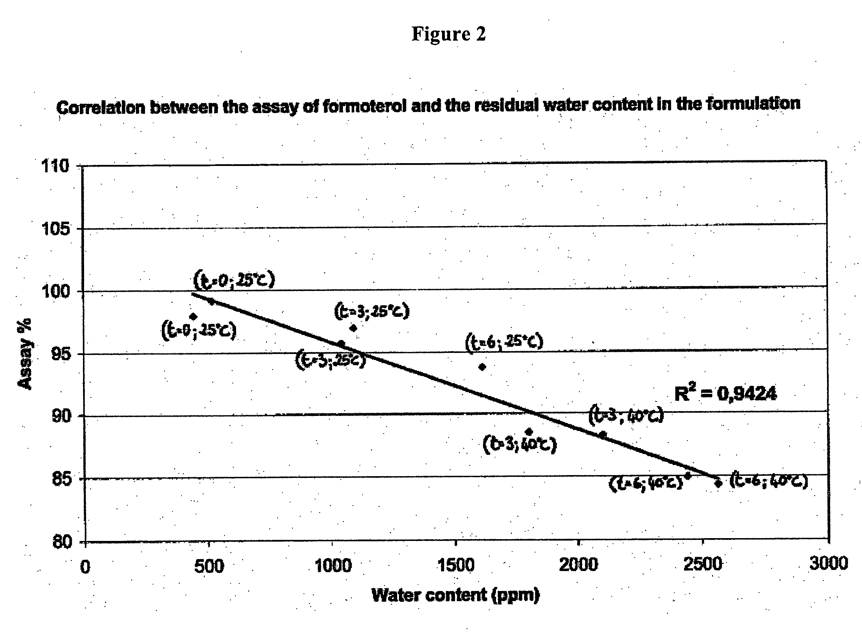

The results, reported in Figure 2, show an inverse linear correlation between the assay of formoterol and the residual amount of water. The numbers between brackets refer to time and temperature condition, respectively. In particular, for a residual humidity higher than 1500 ppm, the assay decreases below 90%. According to pharmaceutical guidelines on stability, loss of 10% of active ingredient does not meet the criteria of acceptance.

## Claims

1. A pharmaceutical formulation to be administered by pMDI which comprises a long acting β₂-agonist as active ingredient in a solution of a liquefied HFA propellant, a co-solvent, optionally 0.05 percent to 5.0 percent of water, **characterised in that** the fraction of particles less than 1.1 µm on actuation of the formulation is higher or equal than 30% as defined by the stages content of the S6-AF of an Andersen Cascade Impactor relative to the respirable dose (stages S3-AF), according to the method referred to in the description on page 23, lines 9-21.

2. A formulation according to claim 1 wherein the active ingredient is selected from formoterol, TA-2005, salmeterol, salts and solvates thereof.

3. A formulation according to claim 2 wherein the active ingredient is formoterol fumarate in a concentration comprised between 0.003 and 0.096% w/v, the amount of water is less than 1500 ppm on the total weight of the formulation, further comprising 0.003% to 0.096% 0.1 M HCl on the total weight of the formulation.

4. A formulation according to claim 3 wherein the pH is comprised between 2.5 and 5.0, preferably between 3.0 and 4.5.

5. A formulation according to claim 2 wherein the active ingredient is TA 2005 hydrochloride in a concentration comprised between 0.0005 and 0.016% w/v and the amount of water is between 0.1 and 0.5% on the total weight of the formulation, further comprising 0.01% to 0.03% 0.1 M HCl on the total weight of the formulation.

6. A formulation according to claim 5 wherein the pH is comprised between 2.5 and 5.0, preferably between 2.8 and 4.0.

7. A formulation according to claim 2 wherein the active ingredient is salmeterol xinafoate in a concentration comprised between 0.02 and 0.05% w/v and the amount of water is comprised between 2% and 5% w/w.

8. A formulation according to any preceding claim filled in a canister having part or all of its internal metallic surfaces made of stainless steel, anodised aluminium or lined with an inert organic coating.

9. A formulation according to claim 8, wherein the canister is lined with an inert organic coating selected from epoxy-phenol resins, perfluoroalkoxyalkane, perfluoroalkoxyalkylene, perfluoroalkylenes such as polytetrafluoroethylene, fluorinated-ethylene-propylene, polyether sulfone and a copolymer fluorinated-ethylene-propylene polyether sulfone.

10. A formulation according to any preceding claim, wherein the propellant includes one or more HFAs selected from the group comprising HFA 134a and HFA 227.

11. A composition according to any preceding claim, wherein the co-solvent is ethanol.

12. A formulation according to claim 11 wherein the concentration of ethanol is comprised between 10 and 30% w/w.

13. A formulation according to claim 3 wherein the co-solvent is anhydrous ethanol.

14. A formulation according to any preceding claim further comprising a further active ingredient selected from the class of steroids such as beclomethasone dipropionate, fluticasone propionate, budesonide and its 22R-epimer or anticholinergic atropine-like derivatives such as ipratropium bromide, oxitropium bromide, tiotropium bromide or drugs useful for the management of respiratory diseases such as methylxanthines, anti-leukotrienes and phosphodiesterase inhibitors.

15. A method of preparing the formulations of claims 1-14, the method comprising:
(a) preparing of a solution of one or more active ingredients in one or more co-solvents;
(b) optionally adding a pre-determined amount of water and/or adjusting the pH of the solution;
(c) filling of the device with said solution;
(d) crimping with valves and gassing;
(e) adding a propellant containing a hydrofluoroalkane (HFA);

16. A pharmaceutical aerosol formulation according to any one of claims 1 to 14 for the treatment of respiratory disorders.

17. A pharmaceutical aerosol formulation according to claim 16 in which the respiratory disorder is asthma or Chronic obstructive pulmonary disease (COPD).

18. A pharmaceutical aerosol formulation according to claim 16 in which the respiratory disease is due to obstruction of the peripheral airways as a result of inflammation or mucus hypersecretion.

19. Use according to claim 16 wherein the respiratory disorder is pulmonary edema or surfactant-deficiency related disorder such as acute lung injury (ALI) or acute respiratory distress syndrome (ARDS).
